# EUROPEAN PATENT SPECIFICATION

(11) **EP 1 089 698 B1**
(45) Date of publication and mention of the grant of the patent: **01.02.2006**
(21) Application number: 99916017.9
(22) Date of filing: 20.04.1999
(51) Int. Cl.: A61L 2/20, A61L 2/26, A61L 2/24, A61H 35/00, A61H 33/14, C02F 1/78, A23L 3/3409

(54) **APPARATUS FOR TREATING ANIMATED OBJECTS WITH OZONE**
VORRICHTUNG ZUR BEHANDLUNG LEBENDER OBJEKTE MIT OZON
APPAREIL DE TRAITEMENT A L'OZONE DES OBJETS VIVANTS

(43) Date of publication of application: 11.04.2001
(73) Proprietor: S.P.M. Recovery Technologies Ltd., 31250 Haifa (IL)
(72) Inventor: PIUK, Vladimir, 29052 Kiryat Yam (IL); SHNAIDERMAN, Mark, 29076 Kiryat Yam (IL)
(74) Representative: Volmer, Johannes Cornelis
(86) International application number: PCT/IL1999/000211
(87) International publication number: WO 2000/062733

(56) References cited:
- EP-A- 0 281 870
- EP-A- 0 450 103
- EP-A- 0 761 237
- WO-A-98/01386
- US-A- 5 000 164
- US-A- 5 029 579
- US-A- 5 312 385
- US-A- 5 447 504
- US-A- 5 662 625

## Description

### FIELD OF THE INVENTION

The present invention relates to apparatus for treating animate objects with ozone.

### BACKGROUND OF THE INVENTION

Ozone is increasingly being used for treating both inanimate objects, such as water supplies, food products, and the like, e.g., for sterilization purposes, as well as animate objects, such as body parts of animals or of human beings for the promotion of healing. The beneficial effects of treatment with ozone in promoting healing are now well recognized. Examples of know ozone-treating methods and apparatus and applications therefor, are described in US Patents 5,052,382 and 5, 098, 415.

EP-A-0761237 is directed to an apparatus wherein compressed air is treated in generator to generate ozonised air which is periodically injected into an enclosure in which materials are sterilised by the ozonised air. The flow of ozonised air into the enclosure is forced by a down stream suction pump and the periodical injection is governed by the valve.

WO 98/01386 teaches an oxygen containing gas which is passed under pressure waves over a product, wherein the embodiments reveal that the apparatus comprises controls for controlling the humidity and temperature in the gas mixture.

EP-A1-0950103 is directed to a medical treatment apparatus using ozone gas, which comprises an airtight body for accommodating a body part therein, a gas forwarding mechanism connected to an ozonizer, and a gas discharge mechanism. The mode of treatment consists of providing a dynamic flow of ozone containing gas over the body part.

Ozone, however, is a dangerous material and can raise a serious health hazard if not handled properly. Thus, a quantity of as little as 0.01 ppm (parts per million) of ozone in the atmosphere can be sensed by human beings, and a concentration of greater than 0.1ppm is regarded as being extremely dangerous. When it is appreciated that ozone treatment processes frequently involve concentrations of as high 50,000 ppm, it will be seen that the smallest leak of ozone to the atmosphere can create a very real health danger to anyone in the immediate vicinity.

### SUMMARY OF THE INVENTION

An object of the present invention is to provide an ozone treatment apparatus, substantially reducing the health hazard present with ozone treatments. Another object of the invention is to provide an apparatus which may be used for administering ozone treatments to animate objects in an efficient and effective manner. According to the invention, an apparatus is provided as defined in claim 1.

The apparatus according to claim 1 is useful for a method of treating an animate object with ozone comprising introducing the object to be treated and ozone into a sealed treatment chamber; and applying a negative pressure to the treatment chamber to prevent ozone leaking from the treatment chamber into the atmosphere and to provide an indication of the condition of the seal.

Furthermore the apparatus according to claim 1 is useful for a method of treating an animate object with ozone comprising, introducing the object to be treated, and a fluid mixture including ozone, into a treatment chamber; and treating the object in the treatment chamber in a static mode under conditions in which there is no flow of ozone into or out of the chamber except that tests are performed at testing intervals wherein, for each test, an outflow from the chamber is produced, the outflow is tested for any drop in ozone content, and a quantity of fresh ozone is introduced into the chamber to make-up for any drop of ozone content therein.

Also, the apparatus according to claim 1 is useful for a method of treating an animate object with ozone, comprising: introducing the object to be treated into a sealed treatment chamber; applying a positive pressure to the treatment chamber; making pressure measurements to determine the drop of positive pressure within the chamber; applying a negative pressure to the chamber; making pressure measurements to determine the drop of negative pressure within the chamber; and if both the positive and negative pressure drops are within predetermined acceptable limits, introducing ozone into the chamber to treat the object therein.

According to the invention, there is provided an apparatus for treating an animate object with ozone, comprising: a sealed treatment chamber for receiving the object, the treatment chamber including an inlet for introducing an ozone mixture therein, and an outlet for removing the ozone mixture therefrom; a supply of ozone connected to the inlet of the treatment chamber; a suction pump connected to the outlet of the treatment chamber; and a control system for controlling the ozone supply and the suction pump to produce a negative pressure in the treatment chamber in order to prevent ozone leakage from the chamber into the atmosphere and to provide an indication of the condition of the seal. Further features of the apparatus according to the invention are apparent from claim 1.

According to a yet further aspect of the invention, there is provided an apparatus for treating an object with ozone, comprising: an air-impermeable housing of a hollow construction to define a treatment chamber, the housing being formed with an inlet and outlet for ozone, and with an opening for introducing the object to be treated into the treatment chamber; a flexible air-impermeable sleeve having one end lining the opening and the opposite end extending externally of the treatment chamber; and a clamping ring clamping the one end of the sleeve to the housing.

As will be described more particularly below, the apparatus of the present invention may be used for administering ozone treatments in a manner which substantially reduces or eliminates the health hazard presented by even extremely low concentrations of ozone in the air. In addition, the apparatus of the present invention can be used for administering ozone in an efficient and effective manner to animate objects for promoting healing, for sterilizing or other purposes.

Further features and advantages of the invention will be apparent from the description below.

### BRIEF DESCRIPTION OF THE DRAWINGS

The invention is herein described, by way of example only, with reference to the accompanying drawings, wherein:
Fig. 1 illustrates one form of treatment chamber constructed in accordance with the present invention for use in apparatus to treating an object with ozone;
Fig. 2 illustrates the overall system to be used with the treatment chamber of Fig. 1; and
Fig. 3 is a flow chart illustrating a preferred mode of operation of the system of Fig. 2.

### DESCRIPTION OF A PREFERRED EMBODIMENT

### The Overall Construction

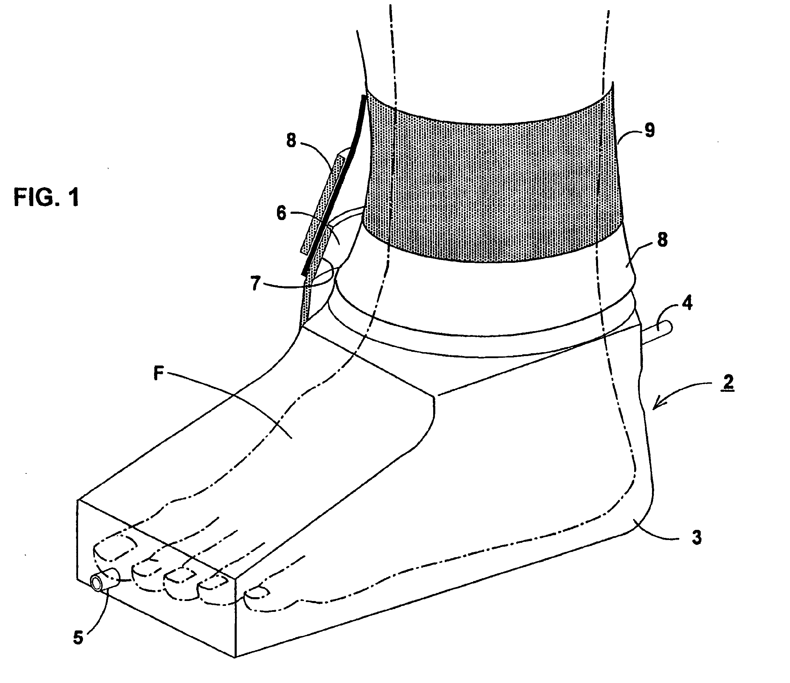

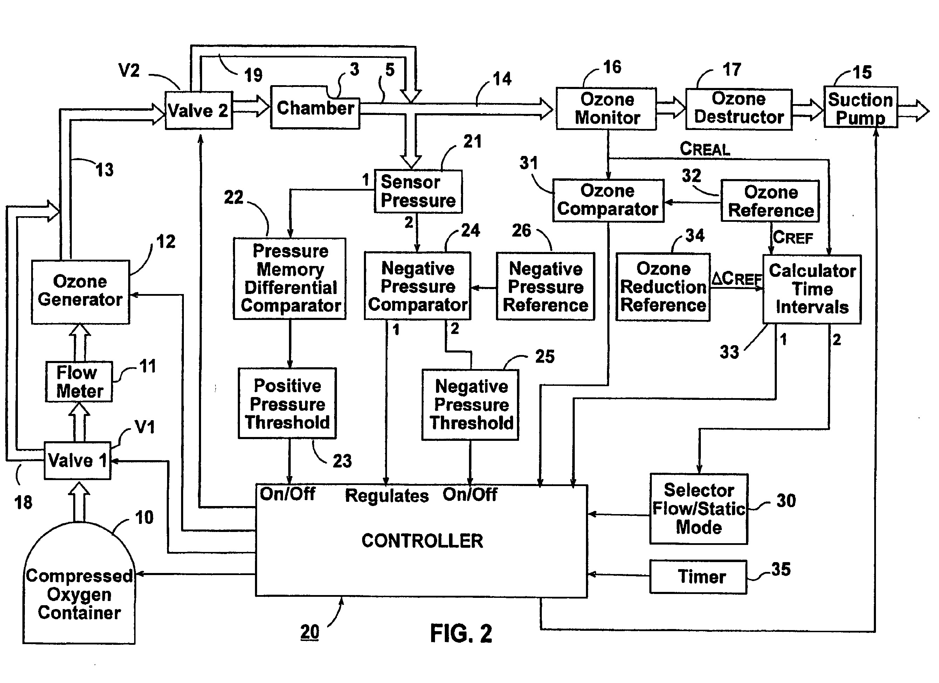

The preferred embodiment of the invention described below is for administering an ozone treatment to a body part, such as a person's foot, in order to promote healing. The apparatus includes a sealed treatment chamber illustrated in Fig. 1 for receiving the object (person's foot) to be treated; and a control system as illustrated in Fig. 2 for supplying the ozone to the treatment chamber and for controlling its delivery in order to make the treatment effective, as well as to decrease the possibility of leakage of ozone to the atmosphere which could create a serious health danger, and to provide an indicator of the condition of the seal.

The apparatus illustrated in Fig. 1, comprises a rigid air-impermeable housing 2 of a hollow construction to define the treatment chamber 3 within it of a configuration to receive the foot F to be treated. Treatment chamber 3 includes an inlet 4 at one end for introducing the ozone, and an outlet 5 at the opposite end for removing the ozone. As described below, the ozone is in the form of a mixture with oxygen. Preferably, the mixture includes 95-98% oxygen and about 2-5% ozone.

Housing 2 is further formed with enlarged opening 6 for receiving the foot F to be treated. Opening 6 is circumscribed by an annular collar 7 having an outer surface of conical configuration complementary to the conical configuration inner surface of a locking ring 8. One end of a flexible, air-impermeable sleeve 9 is interposed between collar 7 and clamping ring 8 and is clamped in place by pressing collar 8 downwardly to produce a friction fit with collar 7. The opposite end of sleeve 9 extends outwardly of housing 2.

Preferably, sleeve 9 is of an elastic material so that the outer end of the sleeve firmly the grips the ankle of the person's foot to be treated, thereby providing a hermetic seal with respect to the treatment chamber 3 within housing 2. Alternatively, sleeve 9 may be of a pliable plastic material, in which case the external end of the sleeve should be firmly clamped against the subject's ankle by the application of the another clamping ring or band (not shown).

### The Ozone Supply and Control System

The supply of the ozone/oxygen mixture to the treatment chamber 3 within housing 2 is shown in Fig. 2. It includes a container of compressed oxygen 10 connected, via a valve V₁ and a flow meter 11, to an ozone generator 12. The ozone generator 12, which may be of a known construction, is connected by a connecting line 13 and a second valve V₂ to the inlet 4 of the treatment chamber 3. The outlet 5 of the treatment chamber is connected by a line 14 to a suction pump 15 for discharge, after the ozone/oxygen mixture has passed through an ozone monitor 16 and an ozone destructor 17.

The ozone supply and control system illustrated in Fig. 2 further includes a bypass line 18 from valve V₁ to the outlet end of the ozone generator 12 for bypassing the ozone generator. A second bypass line 19 connected from valve V₂ to the outlet end of treatment chamber 3 bypasses the treatment chamber.

The overall control is effected by a controller, generally designated 20. As will be described more particularly below, controller 20 controls the suction pump 15, valve V₁, ozone generator 12, and valve V₂. Bypass line 18 and control valve V₁ permit the controller 20 to cause only pure oxygen, or an ozone/oxygen mixture of the required concentration, to be delivered to the treatment chamber 3. Bypass line 19 and control valve V₂ permit controller 20 to measure the pressure drop within chamber 3. This is done by first operating valve V₂ to direct all the flow through the bypass 19 to the outlet end of chamber 3, and then operating valve V₂ to direct all the flow through the chamber 3. These two operations enable measurements to be made of the pressure drop through the chamber.

A pressure sensor 21 is provided at the outlet end of the treatment chamber 3 to measure the pressure thereat. As will be described below, the pressure is measured at the outlet end of chamber 3 during an initial testing mode, to test the seal against leakage from the treatment chamber to the atmosphere, and also during an operational mode, to maintain a pretermined negative pressure within the treatment chamber. The initial testing mode is effected under both positive pressure and negative pressure conditions. Therefore, pressure sensor 21 is connected to the controller 20 via a positive pressure comparator 22 and a positive pressure threshold presetting device 23, and also via a negative pressure comparator 24 and a negative pressure threshold presetting device 25. The negative pressure during the operational mode is preset by negative pressure reference device 26. The outputs of the positive pressure threshold device 23, the negative pressure comparator 24, and the negative pressure threshold device 25, are fed-back to the controller 20.

As will also be described more particularly below, controller 20 may be operated according to a Static operational mode, or a Flow operational mode, as preselected by a mode selector 30. During both operational modes, the ozone concentration in the mixture outletted from treatment chamber 3 is continuously monitored by ozone monitor 16, which supplies feedback signals to controller 20 for controlling the ozone generator 12.

The illustrated system is operated mostly in the Static operational mode. In such a mode, there is no flow into or out of the treatment chamber 3. A normal treatment is generally for a period of 20-30 minutes. Since ozone is relatively unstable, the concentration of the ozone within the treatment chamber continuously diminishes during this treatment period. The rate of diminishment is not easily predetermined since it depends upon many factors, such as temperature, humidity conditions, etc.

Ozone treatment for healing wounds is generally optimum when the ozone concentration is between 2-5% of the ozone/oxygen mixture. Thus, if the treatment starts out with a 5% ozone concentration, during the course of the treatment the concentration could diminish so as to substantially reduce the effectiveness of the treatment. During the Flow operational mode, ozone monitor 16 continuously monitors the ozone concentration, and controls the ozone generator 12 to maintain the optimum ozone/oxygen concentration. However, since there is no flow in the Static operational mode, the ozone concentration cannot be detected by the ozone monitor 16 unless a flow is produced through the outlet of the treatment chamber 3.

As will be described below, such a flow is produced during the Static mode after a predetermined time interval following the start of the treatment, (e.g., 2-5 minutes), and the concentration of ozone is measured. Controller 20 determines the quantity of make-up ozone to be applied to restore the original concentration. The controller also utilizes the magnitude of the make-up quantity to determine the next testing time. Thus, if only a small make-up quantity of ozone is needed to restore the optimum concentration, the next time for testing will be at a longer time interval than if a large make-up quantity of ozone is required. If intervals between tests become too short, controller 20 is automatically switched-over from the Static to the Flow mode.

For accomplishing the latter functions, the illustrated system includes an ozone comparator 31 for comparing the actual ozone concentration (C_{REAL}) as detected by the ozone monitor 16 with a reference concentration (C_{REF}) inputted at 32. The illustrated system further includes a calculator 33 for calculating from C_{REF} (the make-up ozone to restore the original concentration) the time interval for the next testing period, and an ozone reduction reference input device 34 for inputting the initial time interval. The outputs of the ozone comparator 31 and the time interval calculator 33 are fed-back to the controller 20.

### The Operation

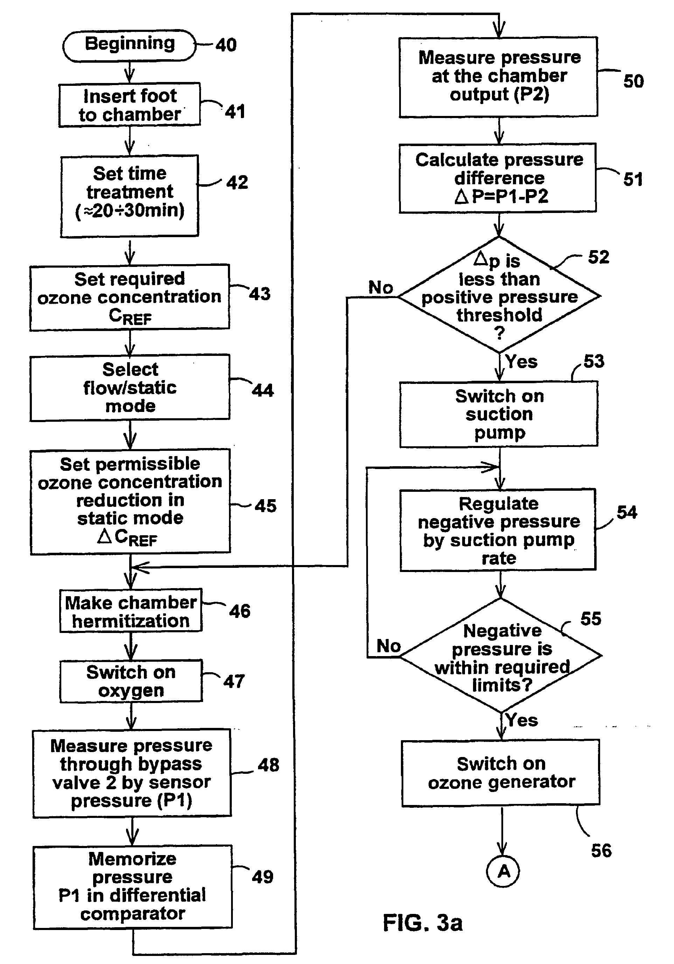

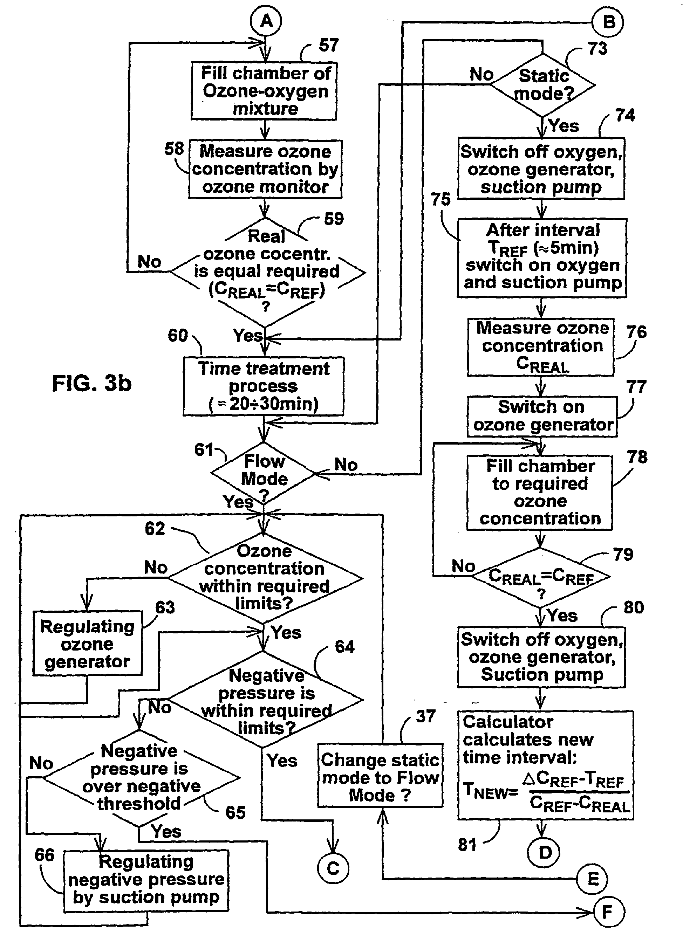

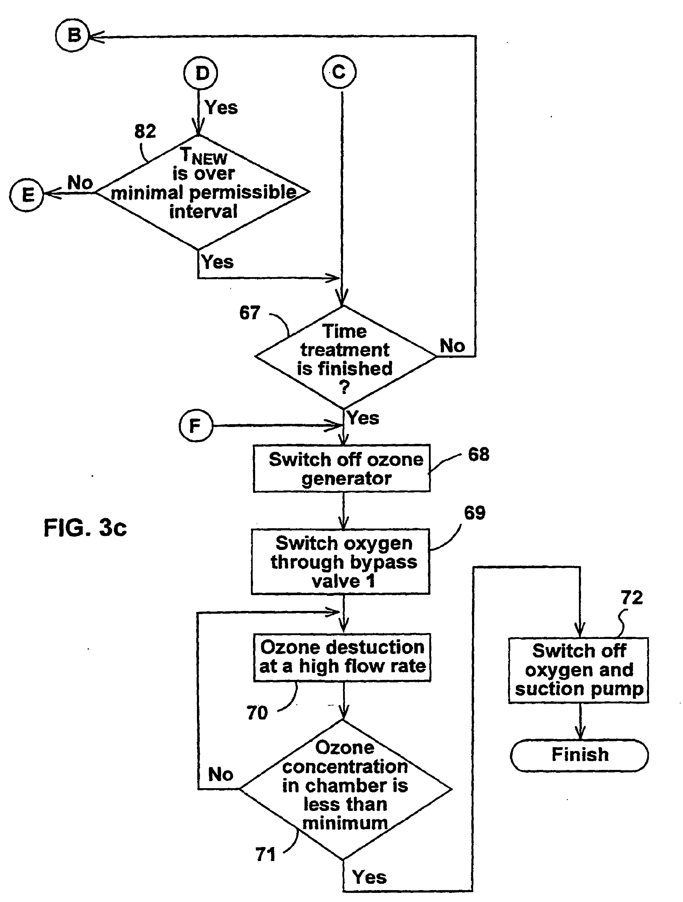

The overall operation of the apparatus illustrated in Figs. 1 and 2 will now be described particularly with reference to the flow charts of Figs. 3a and 3b.

After the subject inserts the foot to be treated into the treatment chamber 3 (block 41), timer 35 of controller 20 is set for the treatment time, e.g. 20-30 minutes (block 42). The desired ozone concentration (C_{ref}) is also preset via reference device 32 (block 43), and the mode selector 30 is then preset for either the Static or Flow mode of operation (block 44). If the Static mode is selected, the permissible reduction in the ozone concentration (C_{ref}) is set via device 34 (block 45).

The treating chamber 3 is then hermetically sealed by securing clamping ring 8 with a friction fit to collar 7 of housing 2. The lower end of the gas-impermeable sleeve 9 is thus clamped between ring 8 and collar 7, and the upper end of the sleeve tightly grips the subject's ankle as shown in Fig. 1. The treatment chamber 3 is thus hermetically sealed (block 46). An initial test of the seal of treating chamber 3 is then made, as follows:

First, valve V₁ is controlled to conduct the oxygen from container 10 towards the treatment chamber 3, bypassing the ozone generator 12 (block 47). Then, valve V₂ is controlled to conduct the oxygen to the outlet end of treatment chamber 3, where it is sensed by pressure sensor 21 (block 48); this provides a measurement of pressure (P₁), corresponding to the pressure at the inlet of the treatment chamber. This pressure is recorded in the memory of comparator 22 (block 49).

Valve V₂ is then controlled to direct the oxygen into the inlet of the treatment chamber 3, and the pressure at the outlet (P₂) is again sensed by pressure sensor 21 and recorded in the memory of comparator 22 (block 50). Controller 20 then calculates the pressure drop (P) experienced in the treatment chamber 3 by the oxygen applied to that chamber under positive pressure (block 51) and determines whether or not P is too high (block 52). If it is too high, this means that the treatment chamber has not been adequately sealed, which requires that the seal be improved and then returning to step 46.

If P is within the permissible threshold, the software makes a negative-pressure check of the seal of the treatment chamber 3 by turning on the suction pump 15, regulating the negative pressure applied by the suction pump to the treatment chamber 3 (block 54), and assuring that the negative pressure is within the required limits (block 55). If so, this indicates that the treatment chamber 3 is adequately sealed, thereby completing the initial testing mode.

When this initial testing mode has been completed, the ozone generator 12 is tumed on (block 56); valve V₁ is controlled to direct the oxygen from container 10 to the ozone generator 12; and valve V₂ is controlled to direct the resulting ozone/oxygen mixture into the treatment chamber 3 (block 57). The ozone concentration at the outlet 5 of the treatment chamber is measured by ozone monitor 16 (block 58), and a determination is made whether the real ozone concentration (C_{REAL}) is equal to that required (C_{REF}) (block 59). If not, the ozone generator 12 is controlled until the required concentration is attained.

The treatment time is then preset by timer 35 (Fig. 2), e.g. for a 20-30 minute treatment period (block 60). A check is made to determine whether the controller is in the Flow or Static mode of operation (block 61).

If in the Flow mode, the ozone/oxygen mixture is continuously supplied to the treatment chamber 3 while the ozone monitor 16 continuously checks the ozone concentration (block 62), and controls the ozone generator 12 (block 63) to maintain the ozone concentration within the required limits. During the Flow mode, the pressure sensor 21 also senses negative pressure at the outlet of the treatment chamber 3 and checks whether the negative pressure is within the required limits (block 64), and is over the negative pressure threshold (block 65) fixed by the negative pressure threshold device 25. Controller 20 controls the suction pump 15 (block 66) to maintain the negative pressure within the required limits, preferably 5-10 mm Hg below atmosphere.

The foregoing Flow mode of operation is continued until the preset treatment time is completed (block 67), whereupon the ozone generator 12 is switched off (block 68), valve V₁ is actuated to conduct the oxygen through the bypass 18 and through the treating chamber 3 to exhaust the ozone within that chamber (block 69), and the ozone destructor 17 is actuated at a high rate (block 70) to convert the ozone to oxygen at a high rate. This continues until ozone monitor 16 senses that the ozone within in the treatment chamber 3 is below a predetermined minimum (block 71), whereupon valve V₁ is actuated to switch-off the supply of oxygen, and the suction pump 15 is tumed-off by the controller (block 72).

If when the mode check was made (block 61), it was determined that the controller is in the Static mode (block 73), the oxygen the ozone generator 12, and the suction pump 15 are all switched off (block 74), and the object within the treatment chamber 3 is subjected to a static ozone/oxygen treatment.

After a predetermined time interval (e.g. 2-5 minutes), a test is made to determine the ozone concentration within the treatment chamber 3 to assure that the ozone concentration has not unduly dropped because of its unstability and is still within the prescribed range (C_{REF}). To make this test, valve V₁ is controlled to direct the oxygen flow through the ozone generator 12 and into the treatment chamber 3 while the suction pump 15 is energized. The ozone concentration is at the outlets of the treatment chamber is measured by ozone monitor 16 (block 76). The ozone generator 12 is switched-on (block 77), to fill the treatment chamber 3 with make-up ozone/oxygen mixture until the concentration is found to be within the prescribed limits (block 79), at which time the oxygen, ozone generator, and suction pump are switched off (block 80). A calculation is then made to determine the time interval for the next time for testing according to the make-up ozone added to the treatment chamber 3 (block 81).

The foregoing Static mode treatment is continued, and the next time interval for testing is computed according to the amount of make-up ozone supplied, as described above. Whenever the next time for testing is found to be below a predetermined value, the controller automatically switches-over to the Continuous mode of treatment (block 82).

While the invention has been described with respect to one preferred embodiment, it will be appreciated that this is set forth merely for purposes of example, and that many other variations, modifications and applications of the invention may be made.

## Claims

1. Apparatus for treating an animate object with ozone, comprising:
a treatment chamber (3) for receiving the animate object, said treatment chamber (3) including an inlet (4) for introducing an
ozone-oxygen mixture therein, and an outlet (5) for removing the ozone-oxygen mixture therefrom;
sealing means (7, 8) for hermetically sealing the treatment chamber (3);
a supply of ozone-oxygen connected to the inlet (4) of said treatment chamber (3);
a suction pump (15) connected to the outlet (5) of said treatment chamber (3);
a control system for controlling said supply of ozone-oxygen and said suction pump (15) to produce a negative pressure in said treatment chamber (3) in order to prevent ozone leakage from the chamber (3) into the atmosphere and to provide an indication of the condition of the seal;
wherein said control system is a means for producing a static mode of operation which is defined as conditions under which there is no flow of ozone into or out of the chamber except that tests are performed at testing intervals wherein an outflow from the chamber is produced, the outflow is tested for any drop in ozone content, and a quantity of fresh ozone is introduced into the chamber to make up for any drop of ozone content therein;
wherein said supply of ozone-oxygen includes a source of oxygen (10), an ozone generator (12) connected to the source (10) of oxygen to receive oxygen and to generate ozone therefrom, a connecting line (13) connecting the outlet of the ozone generator (12) to the inlet (4) of said treatment chamber (3), and a bypass line (18) bypassing said ozone generator (12) and connecting said source (10) of oxygen directly to said connecting line (13);
wherein said control system further includes a valve (V₁), said valve (V₁) being controlled by a controller (20) for selectively directing the oxygen from said source (10) of oxygen either to the inlet to said ozone generator (12) or to the outlet from said ozone generator (12) via said bypass line (18);
wherein said supply of ozone-oxygen further includes a second bypass line (19) connected to said connecting line (13) and bypassing said treatment chamber(3);
wherein said control system further includes a second valve (V₂) controlled by said controller (20) for selectively directing the ozone-oxygen mixture to the inlet (4) or to the outlet (5) of said treatment chamber (3).

2. Apparatus according to claim 1, wherein the treatment chamber (3) is defined by an air-impermeable housing (2) of a hollow construction, said housing (2) having an inlet (4) and an outlet (5) for ozone, and an opening (6) for introducing an animate object to be treated, in the treatment chamber (3);
a flexible air-impermeable sleeve (9) having one end lining the opening (6) and an opposite end extending externally to the treatment chamber (3), a clamping ring (8) clamping one end of the flexible sleeve (9) to the housing (2), and wherein
the inner surface of the clamping ring (8) has a conical configuration, said clamping ring (8) being receivable with a friction fit over a complementary conical section of the housing (2), such that one end of the flexible sleeve (9) is clamped between the conical ring (8) and the conical section of the housing (2).

3. Apparatus according to one of the preceding claims, wherein the control system includes a controller (20) programmed to measure the magnitude of the make-up ozone introduced into the treatment chamber (3) a pressure sensor (21) for sensing the pressure at the outlet (5) from said treatment chamber (3) and for imputing a control signal to said controller (20) in response thereto.

4. Apparatus according to claim 3, wherein the control system further includes an ozone monitor (16) for sensing the ozone at the outlet (5) of said treatment chamber (3) and for imputing another control signal to said controller (20) in response thereto.

5. Apparatus according to claim 4, including a pressure memory differential comparator (22), which comparator (22) is capable of calculating the pressure difference at the inlet (4) and at the outlet (5) of the treatment chamber (3).

## Patentansprüche

1. Vorrichtung zur Behandlung eines lebenden Objekts mit Ozon, umfassend:
eine Behandlungskammer (3) zum Aufnehmen des lebenden Objekts, wobei die Behandlungskammer (3) einen Einlass (4), um ein Ozon-Sauerstoff-Gemisch darin einzuführen, und einen Auslass (5), um das Ozon-Sauerstoff-Gemisch daraus zu entfernen, umfasst;
Dichtungsmittel (7, 8) zum hermetischen Abdichten der Behandlungskammer (3);
eine Ozon-Sauerstoff-Zuleitung, die an den Einlass (4) der Behandlungskammer (3) angeschlossen ist;
eine Absaugpumpe (15), die an den Auslass (5) der Behandlungskammer (3) angeschlossen ist;
ein Regelungssystem, um die Ozon-Sauerstoff-Zuleitung und die Absaugpumpe (15) zu regeln, um einen negativen Druck in der Behandlungskammer (3) zu erzeugen, um ein Ausströmen von Ozon aus der Kammer (3) in die Atmosphäre zu verhindern und eine Angabe des Zustands der Dichtung bereitzustellen;
wobei das Regelungssystem ein Mittel zum Erzeugen eines statischen Betriebsmodus ist, der definiert wird als Bedingungen, unter denen kein Ozonfluss in die oder aus der Kammer erfolgt, außer dass Prüfungen in Prüfintervallen durchgeführt werden, wobei ein Abfluss aus der Kammer erzeugt wird, der Abfluss auf einen Abfall des Ozongehalts geprüft wird, und eine Menge von frischem Ozon in die Kammer eingeführt wird, um einen Abfall des Ozongehalts darin auszugleichen;
wobei die Ozon-Sauerstoff-Zuleitung eine Sauerstoffquelle (10), einen Ozon-Generator 12, der an die Sauerstoffquelle (10) angeschlossen ist, um Sauerstoff aufzunehmen und daraus Ozon zu erzeugen, eine Verbindungsleitung (13), die den Auslass des Ozon-Generators (12) mit dem Einlass (4) der Behandlungskammer (3) verbindet, und eine Umgehungsleitung (18), die den Ozon-Generator (12) umgeht und die Sauerstoffquelle (10) direkt mit der Verbindungsleitung (13) verbindet, umfasst;
wobei das Regelungssystem des Weiteren ein Ventil (V₁) umfasst, wobei das Ventil (V₁) durch eine Steuereinrichtung (20) gesteuert wird, um den Sauerstoff aus der Sauerstoffquelle (10) selektiv entweder zu dem Einlass des Ozon-Generators (12) oder zu dem Auslass aus dem Ozon-Generator (12) über die Umgehungsleitung (18) zu leiten;
wobei die Ozon-Sauerstoff-Zuleitung des Weiteren eine zweite Umgehungsleitung (19) umfasst, die an die Verbindungsleitung (13) angeschlossen ist und die Behandlungskammer (3) umgeht;
wobei das Regelungssystem des Weiteren ein zweites Ventil (V₂) umfasst, das durch die Steuereinrichtung (20) gesteuert wird, um das Ozon-Sauerstoff-Gemisch zu dem Einlass (4) oder zu dem Auslass (5) der Behandlungskammer (3) zu leiten.

2. Vorrichtung nach Anspruch 1, wobei die Behandlungskammer (3) durch ein luftundurchlässiges Gehäuse (2) aus einer hohlen Konstruktion definiert wird, wobei das Gehäuse (2) einen Einlass (4) und einen Auslass (5) für Ozon und eine Öffnung (6) zum Einführen eines zu behandelnden lebenden Objekts in die Behandlungskammer (3) aufweist;
wobei eine biegsame luftundurchlässige Manschette (9) ein Ende, das die Öffnung (6) umsäumt, und ein gegenüberliegendes Ende, das sich nach außen zu der Behandlungskammer (3) erstreckt, einen Klemmring (8), der ein Ende der biegsamen Manschette (9) an das Gehäuse (2) klemmt, aufweist, und wobei die Innenfläche des Klemmrings (8) eine konische Auslegung aufweist, wobei der Klemmring (8) mit einer Reibungspassung über einem komplementären konischen Abschnitt des Gehäuse (2) aufgenommen werden kann, so dass ein Ende der biegsamen Manschette (9) zwischen den konischen Ring (8) und den konischen Abschnitt des Gehäuses (2) geklemmt wird.

3. Vorrichtung nach einem der vorhergehenden Ansprüche, wobei das Regelungssystem eine Steuereinrichtung (20), die so programmiert ist, dass sie die Größe des in die Behandlungskammer (3) eingeführten Ausgleichs-Ozons misst, einen Drucksensor (21) zum Erfassen des Drucks an dem Auslass (5) aus der Behandlungskammer (3) und zum Zuweisen eines Steuersignals zu der Steuereinrichtung (20) in Reaktion darauf aufweist.

4. Vorrichtung nach Anspruch 3, wobei das Regelungssystem des Weiteren einen Ozon-Monitor (16) zum Erfassen des Ozons an dem Ausgang (5) der Behandlungskammer (3) und zum Zuweisen eines weiteren Steuersignals zu der Steuereinrichtung (20) in Reaktion darauf umfasst.

5. Vorrichtung nach Anspruch 4, umfassend einen Druckspeicher-Differenzvergleicher (22) (pressure memory differential comparator), welcher Vergleicher (22) in der Lage ist, die Druckdifferenz an dem Einlass (4) und an dem Auslass (5) der Behandlungskammer (3) zu berechnen.

## Revendications

1. Appareil pour traiter un objet vivant avec de l'ozone, comprenant :
une chambre de traitement (3) pour recevoir l'objet vivant, ladite chambre de traitement (3) comprenant une admission (4) pour introduire un mélange d'ozone et d'oxygène dans celle-ci, et une sortie (5) pour évacuer le mélange d'ozone et d'oxygène à partir de celle-ci ;
des moyens de fermeture (7, 8) pour fermer hermétiquement la chambre de traitement (3) ;
une alimentation en ozone et oxygène raccordée à l'admission (4) de ladite chambre de traitement (3) ;
une pompe d'aspiration (15) raccordée à la sortie (5) de ladite chambre de traitement (3) ;
un système de commande pour commander ladite alimentation en ozone et oxygène et ladite pompe d'aspiration (15) pour produire une pression négative dans ladite chambre de traitement (3) afin d'éviter une fuite d'ozone à partir de la chambre (3) dans l'atmosphère et de fournir une indication de la condition de la fermeture ;
dans lequel ledit système de commande est un moyen destiné à produire un mode statique de fonctionnement qui est défini comme des conditions dans lesquelles il n'y a pas d'écoulement d'ozone dans ou hors de la chambre, sauf lorsque des essais sont réalisés à des intervalles d'essai lors desquels un écoulement à partir de la chambre est produit, l'écoulement est soumis à essai en ce qui concerne une quelconque chute de la teneur en ozone, et une quantité d'ozone frais est introduite dans la chambre pour pallier une quelconque chute de la teneur en ozone dans celle-ci ;
dans lequel ladite alimentation en ozone et oxygène comprend une source d'oxygène (10), un générateur d'ozone (12) raccordé à la source (10) d'oxygène pour recevoir l'oxygène et pour générer de l'ozone à partir de celui-ci, une conduite de liaison (13) raccordant la sortie du générateur d'ozone (12) à l'admission (4) de ladite chambre de traitement (3), et une conduite de contournement (18) contournant ledit générateur d'ozone (12) et raccordant ladite source (10) d'oxygène directement à ladite conduite de liaison (13) ;
dans lequel ledit système de commande comprend en outre une valve (V₁), ladite valve (V₁) étant commandée par un dispositif de commande (20) pour diriger de façon sélective l'oxygène à partir de ladite source (10) d'oxygène soit vers l'admission vers ledit générateur d'ozone (12), soit vers la sortie à partir dudit générateur d'ozone (12) par l'intermédiaire de ladite conduite de contournement (18) ;
dans lequel ladite alimentation en ozone et oxygène comprend en outre une seconde conduite de contournement (19) raccordée à ladite conduite de liaison (13) et contournant ladite chambre de traitement (3) ;
dans lequel ledit système de commande comprend en outre une seconde valve (V₂) commandée par ledit dispositif de commande (20) pour diriger de façon sélective le mélange d'ozone et d'oxygène vers l'admission (4) ou la sortie (5) de ladite chambre de traitement (3).

2. Appareil selon la revendication 1, dans lequel la chambre de traitement (3) est définie par un boîtier imperméable à l'air (2) d'une construction creuse, ledit boîtier (2) comportant une admission (4) et une sortie (5) pour l'ozone, et une ouverture (6) pour introduire un objet vivant devant être traité, dans la chambre de traitement (3) ;
un manchon souple imperméable à l'air (9) comportant une extrémité garnissant l'ouverture (6) et une extrémité opposée s'étendant de façon externe sur la chambre de traitement (3), une bride de serrage (8) serrant une extrémité du manchon souple (9) sur le boîtier (2), et dans lequel
la surface interne de la bride de serrage (8) a une configuration conique, ladite bride de serrage (8) pouvant être reçue avec un ajustement serré sur une partie conique complémentaire du boîtier (2), de telle sorte qu'une extrémité du manchon souple (9) est serrée entre la bride conique (8) et la partie conique du boîtier (2).

3. Appareil selon l'une des revendications précédentes, dans lequel le système de commande comprend un dispositif de commande (20) programmé pour mesurer la quantité de l'ozone d'appoint introduit dans la chambre de traitement (3), un capteur de pression (21) pour détecter la pression au niveau de la sortie (5) à partir de ladite chambre de traitement (3) et pour envoyer un signal de commande audit dispositif de commande (20) en réponse à celui-ci.

4. Appareil selon la revendication 3, dans lequel le système de commande comprend en outre un dispositif de surveillance de l'ozone (16) pour détecter l'ozone au niveau de la sortie (5) de ladite chambre de traitement (3) et pour envoyer un autre signal de commande audit dispositif de commande (20) en réponse à celui-ci.

5. Appareil selon la revendication 4, comprenant un comparateur différentiel à mémoire de pression (22), lequel comparateur (22) est capable de calculer la différence de pression au niveau de l'admission (4) et au niveau de la sortie (5) de la chambre de traitement (3).
